# EUROPEAN PATENT APPLICATION

(11) **EP 2 272 874 A1**
(43) Date of publication of application: **12.01.2011**
(21) Application number: 09009063.0
(22) Date of filing: 10.07.2009
(51) Int. Cl.: C07K 16/40, C12N 9/20, A61K 39/395, G01N 33/50

(54) **Role of PLD1 in thrombus formation and integrin alpha IIb beta 3 activation**

(71) Applicant: CSL Behring GmbH, 35041 Marburg (DE); Julius-Maximilians-Universität Würzburg, 97070 Würzburg (DE)
(72) Inventor: Nieswandt, Bernhard, 97246 Eibelstadt (DE); Stoll, Guido, 97222 Rimpar (DE)

(57) **Abstract**

The present invention relates to an inhibitor of phospholipase D1 (PLD1) or an inhibitor of an activator of PLD1 for use in treating or preventing a disorder related to venous or arterial thrombus formation or as a lead compound for developing a drug for treating or preventing a disorder related to venous or arterial thrombus formation. The present invention also relates to a method of treating and/or preventing a disorder related to venous or arterial thrombus formation comprising administering a pharmaceutically effective amount of an inhibitor of phospholipase D1 (PLD1) or an inhibitor of an activator of PLD1 to a subject in need thereof. The present invention further relates to methods of identifying a compound suitable as a lead compound and/or as a medicament for the treatment and/or prevention of a disorder related to venous or arterial thrombus formation.

## Description

The present invention relates to an inhibitor of phospholipase D1 (PLD1) or an inhibitor of an activator of PLD1 for use in treating or preventing a disorder related to venous or arterial thrombus formation or as a lead compound for developing a drug for treating or preventing a disorder related to venous or arterial thrombus formation. The present invention also relates to a method of treating and/or preventing a disorder related to venous or arterial thrombus formation comprising administering a pharmaceutically effective amount of an inhibitor of phospholipase D1 (PLD1) or an inhibitor of an activator of PLD1 to a subject in need thereof. The present invention further relates to methods of identifying a compound suitable as a lead compound and/or as a medicament for the treatment and/or prevention of a disorder related to venous or arterial thrombus formation.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Adhesion and activation of platelets at sites of vascular injury is essential to limit posttraumatic blood loss but is also a major pathomechanism underlying acute ischemic cardio- and cerebrovascular events¹. Therefore, inhibition of platelet function is an important strategy for prevention and treatment of myocardial infarction² and possibly stroke^{2;3}. Under conditions of elevated shear as found in arterioles or stenosed arteries, platelet recruitment to the ECM is initiated by the rapid but reversible interaction between glycoprotein (GP)Ib-V-IX and collagen-bound von Willebrand factor (vWF)⁴. For stable adhesion to occur, intracellular signals are required that lead to conformational changes within the integrin adhesion receptors, most notably integrin αllbβ3, to allow efficient ligand binding mediated by a process referred to as "inside-out" activation. Under flow, GPIb-vWF interactions can elicit such intracellular signals and weak αllbβ3 activation^{5;6}, but the underlying mechanisms have remained elusive. Although collagens, thromboxane A₂ (TxA₂) and ADP released from activated platelets, and locally produced thrombin contribute to full platelet activation at sites of injury through different signaling pathways⁷, a recent study has suggested that platelet aggregate formation is driven mainly by shear forces⁸, most likely through a GPlb-triggered process. All these signaling pathways activate phospholipases (PL), which cleave membrane phospholipids to generate intracellular second messengers. PLC has the best defined role in platelet activation and integrin regulation⁹. PLC activity generates inositol 1,4,5-triphosphate (IP₃) which triggers Ca²⁺ mobilization, and diacylglycerol (DAG), which in combination with Ca²⁺ activates the small GTPase guananine exchange factor CaIDAG-GEFI¹⁰, leading to activation of the small GTPase Rap1¹¹, which directly controls inside-out activation of αllbβ3 through a pathway involving multiple proteins, including talin, that bind to the cytoplasmic tail of integrin¹²⁻¹⁵. The talin interaction is faciliated by the pleiotropic lipid phosphoinositol 4,5 bisphosphate (PI4,5P₂)¹⁶.

Agonist stimulation can independently generate DAG through phosphorylation of phosphatidic acid (PA), a signaling lipid produced by the enzyme Phospholipase D (PLD)^{17;18} and PA itself directly stimulates synthesis of PI4,5P₂¹⁹. Platelets express the two classical isoforms, PLD1 and PLD2, which translocate to the plasma membrane during platelet activation and produce PA²⁴. However, lacking genetic tools, the function of PLD in the process of platelet activation and aggregation has remained poorly explored.

Despite the fact that thrombus formation leads to some of the most frequently occurring diseases in humans und despite extensive basic and clinical research that has been carried out in the field of thrombosis over decades, medicaments that have been registered and are presently available for patients are unsatisfactory for a variety of reasons. One problem common to all anti-coagulants presently used in clinics is their association with an increased risk of serious bleeding. These include heparins, Cumarins, direct thrombin inhibitors such as hirudin, as well as aspirin, P2Yi2 inhibitors such as Clopidogrel and GPIIb/IIIa inhibitors such as abciximab (ReoPro). In addition, for some of these known inhibitors such as aspirin or the P2Yi2 inhibitor Clopidogrel, many patients have been described as low or nonresponders (23).

The technical problem underlying the present invention is thus the provision of alternative and/or improved means and methods for treating diseases based on thrombus formation.

The solution to this technical problem is achieved by providing the embodiments characterised in the claims.

Accordingly, the present invention relates to an inhibitor of phospholipase D1 (PLD1) or an inhibitor of an activator of PLD1 for use in treating or preventing a disorder related to venous or arterial thrombus formation or as a lead compound for developing a drug for treating or preventing a disorder related to venous or arterial thrombus formation.

The term "PLD1" relates to "phospholipase D1" and both terms are used interchangeably herein. PLD1 (GeneID: 5337; two isoforms are known, the first having GenBank database accession no. NP_002653.1 and UniProtKB accession number Q13393; the second having GenBank database accession no. NP_01123553.1 and UniProtKB accession number Q59EA4). PLDs are phosphatidylcholine-specific phospholipases D, with the EC number 3.1.4.4. PLDs catalyse the hydrolysis of phosphatidylcholine to produce phosphatidic acid and choline. A range of agonists acting through G protein-coupled receptors and receptor tyrosine kinases stimulate this hydrolysis. Phosphatidylcholine-specific PLD activity has been implicated in numerous cellular pathways, including signal transduction, membrane trafficking, and the regulation of mitosis as well as chemotaxis²⁰ and cell migration²¹.

The term "inhibitor" in accordance with the present invention refers to an inhibitor that reduces the biological function of a particular target protein. An inhibitor may perform any one or more of the following effects in order to reduce the biological function of the protein to be inhibited: (i) the transcription of the gene encoding the protein to be inhibited is lowered, i.e. the level of mRNA is lowered, (ii) the translation of the mRNA encoding the protein to be inhibited is lowered, and (iii) the protein performs its biochemical and/or cellular function with lowered efficiency in the presence of the inhibitor.

Compounds failing in class (i) include compounds interfering with the transcriptional machinery and/or its interaction with the promoter of said gene and/or with expression control elements remote from the promoter such as enhancers. Preferably, said compounds directly lower the transcription of PLD1, i.e. the mRNA level of PLD1 is lowered as a direct consequence of the presence of the compound. Compounds of class (ii) comprise antisense constructs and constructs for performing RNA interference (e.g. siRNA, shRNA, miRNA) well known in the art (see, e.g. Zamore (2001) Nat. Struct. Biol. 8(9), 746; Tuschl (2001) Chembiochem. 2(4), 239). Preferably, these compounds directly lower the translation of PLD1 mRNA, e.g. in the case of siRNA by specifically binding to the mRNA of PLD1. Compounds of class (iii) interfere with the molecular function of the protein to be inhibited, in the present case of the PLD1 activation as well as its down-stream signalling activity and its activity on platelet aggregation. Accordingly, active site binding compounds, in particular compounds capable of directly binding, and inhibiting, PLD1 are preferred. Class (iii) also includes compounds which do not necessarily bind directly to PLD1, but still interfere with PLD1 activity, for example by binding to and/or inhibiting the function or expression of members of a pathway which comprises PLD1. Preferably, these members are downstream of PLD1 within said pathway.

The inhibitor, in accordance with the present invention, can in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Method for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to elevate the level of the encoded inhibitor in any cell or a subset of selected cells of the recipient either permanently or for a specific time period. Thus, a tissue- and/or time-dependent expression of the inhibitor can be achieved, for example restricted to blood cells. Thus, in a preferred embodiment, the inhibitor is a thrombocyte-specific inhibitor.

The term "inhibitor of PLD1" in accordance with the present invention refers to an inhibitor that reduces the biological function of PLD1, Biological function denotes in particular any known biological function of PLD1 including functions elucidated in accordance with the present invention. Examples of said biological function are thrombus formation, e.g. formation of stable platelet-platelet contacts under high shear flow conditions, fibrinogen binding or thrombocyte adhesion to von Willebrand factor. These functions can be tested for either using any of a variety of standard methods known in the art, such as for example standard aggregometry, *ex vivo* flow adhesion studies, flow cytometric methods for determination of platelet activation, *in vivo* thrombosis models in animals or on the basis of the teachings of the examples provided below, optionally in conjunction with the teachings of the documents cited therein.

In a preferred embodiment, the inhibitor reduces the biological function of PLD1 by at least 50%, preferably by at least 75%, more preferred by at least 90% and even more preferred by at least 95% such as at least 98% or even by 100%. The term reduction by at least, for example 75%, refers to a decreased biological function such that PLD1 looses 75% of its function and, consequently, has only 25% activity remaining as compared to PLD1 that is not inhibited. The efficiency of the inhibitor can be quantified by comparing the level of activity in the presence of the inhibitor to that in the absence of the inhibitor.

The term "inhibitor of an activator of PLD1" refers to inhibitors that do not directly interact with PLD1 but with molecules that directly or indirectly activate one or more of the biological functions of PLD1 and preferably one or more of those functions referred to above or elsewhere in this specification. Non-limiting examples of molecules that directly or indirectly activate one or more of the biological functions of PLD1 include molecules that are upstream of PLD1 in a signal transduction pathway. The inhibition values referred to above for inhibitors of PLD1 mutatis mutandis apply to inhibitors of an activator of PLD1. Preferably, the activator of PLD1 is not GPIb-V-IX, GPVI, protease activated receptors 1 and/or 4, ADP receptors P2Y1 and P2Y12, CLEC-2 or thromboxane A2 receptor. Instead, the activator preferably is a molecule that is downstream of these receptors in the signaling pathway that leads to activation of PLD1.

The function of any of the inhibitors referred to in the present invention may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of binding of potential inhibitors can be effected in, for example, any binding assay, preferably biophysical binding assay, which may be used to identify binding test molecules prior to performing the functional/activity assay with the inhibitor. Suitable biophysical binding assays are known in the art and comprise fluorescence polarisation (FP) assay, fluorescence resonance energy transfer (FRET) assay and surface plasmon resonance (SPR) assay.

In cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidiumbromide or SybrGreen. A lower band intensity of the sample treated with the inhibitor as compared to a non-treated sample indicates that the inhibitor successfully inhibits the expression of the protein on the nucleic acid level.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

Methods for the determination of the expression of a protein on the amino acid level include but are not limited to western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognising the protein of interest. After washing, a second antibody specifically recognising the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique.

The term "disorder related to venous thrombus formation" as used herein relates to diseases caused by or related to blood clot formation within a vein. The term "disorder related to arterial thrombus formation" as used herein relates to diseases caused by or related to the formation of a thrombus within an artery. The disorder related to venous or arterial thrombus formation is selected from, but not limited to, the group consisting of myocardial infarction, stroke, ischemic stroke, pulmonary thromboembolism, peripheral artery disease (PAD), arterial thrombosis and venous thrombosis.

Where the inhibitor is used as a lead compound for the development of a drug for treating or preventing a disorder related to venous or arterial thrombus formation, the following developments are considered: modifications to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiff's bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000). Those lead compounds will also allow for the development of novel, highly effective, yet safe antithrombotics.

In accordance with the present invention it was surprisingly found that PLD1 is a critical regulator of platelet activity in the setting of ischemic cardio- and cerebrovascular events. As shown herein, PLD1 deficiency leads to impaired integrin αllbβ3 inside-out signaling and instable thrombus formation, particularly under high shear flow conditions. These defects result in protection against arterial thrombosis and ischemic brain infarction, demonstrating that PLD1 is a fundamental mediator in thrombotic processes. PLD has been implicated in facilitating many elementary cell functions, including endocytosis and exocytosis (degranulation), cytoskeletal reorganization, and cell proliferation and migration^{17;18}. However, mice lacking PLD1 are viable, healthy and fertile, suggesting that PLD1 deficiency can be functionally compensated by the actions of the other classical isoform, PLD2, or any of the many other signaling enzymes that cells can use to increase the production or decrease the catabolism of PA, such as DAG kinases, LysoPA acetyltransferases, and PA phosphatases.

The findings presented herein clearly show a requirement for PLD1 and PA in G-protein- and ITAM-dependent integrin αllbβ3 activation and, in addition, reveal a central function of the enzyme in GPlb-triggered signaling events that allow platelet adhesion under high shear flow and pathophysiological conditions. In contrast, no requirement for PLD1 in the process of degranulation of platelet alpha-granules was found.

In accordance with the present invention it was shown for *Pld1*^{*-*/*-*} platelets, that recruitment and initial attachment to collagen, a process known to be driven mainly by GPlb, GPVI and integrins, appeared to be normal, whereas subsequent stable platelet-platelet interaction was defective. This indicates that the first layer of platelets is sufficiently activated, presumably through GPVI-collagen interaction, to firmly attach to the matrix, whereas in upper layers of the growing thrombus, platelet activation was impaired. Activation of newly recruited platelets on the surface of adherent platelets relies on signals derived from GPIb-vWF interactions and released secondary mediators, most notably ADP and TxA2. The data presented herein show that lack of PLD1 affects this activation process, particularly under high shear flow conditions, strongly suggesting a role of PLD1 in signaling events downstream of GPlb that lead to αllbβ3 activation. Furthermore, this defect also extends to the function of platelets in stimulating the coagulation process, since *Pld1*^{*-*/*-*} platelets showed a marked reduction in collagen-vWF-dependent phosphatidylserine exposure under shear condition, which is known to be facilitated by GPlb and αIIbβ3 activation²⁷.

The unaltered bleeding times in *Pld1*^{*-*/*-*} mice make PLD1 a promising target for antithrombotic agents not being associated with increased bleeding, in contrast to anti-coagulants presently used in clinics. This is of particular interest for the treatment or prophylaxis of ischemic stroke where the risk of intracranial bleeding is a major limitation of current antithrombotic treatment³⁰. Blockade of pathologic platelet activity and platelet-receptor-ligand interactions has demonstrated that platelets play a fundamental role in cerebral ischemic events, one of the leading causes of death in industrialized countries³¹. It has recently been shown that the inhibition of GPlb or GPVI efficiently ameliorated lesion formation in the tMCAO model of ischemic stroke. While this approach did not induce intracranial hemorrhage, increased bleeding times were nonetheless observed for the animals²⁸. In contrast, while the protection from lesion formation seen in *Pld1*^{*-*/*-*} mice was comparable to that achieved by GPlb inhibition (>70% reduction in infarct size²⁸), hemostasis was not affected. This indicates that despite the lack of a clear correlation between bleeding time and risk of clinical hemorrhages³², PLD1 or targets downstream of PLD1 action might be a preferable target for safe inhibition of thrombotic activity, in particular in the setting of ischemic stroke. Furthermore, targets activating PLD1 but being downstream of GPlb, GPVI, protease activated receptors 1 and/or 4, ADP receptors P2Y1 and P2Y12, CLEC-2 or thromboxane A2 receptor may also provide a suitable target for safe inhibition of thrombotic activity, in particular in the setting of ischemic stroke. In addition, PLD1 deficiency was not associated with any obvious developmental defects, fertility or impairment of any vital functions. Taken together, these findings show that inhibitors of PLD1 provide a novel therapeutic opportunity for prevention and treatment of thromboembolic events in the arterial system.

Preferably, the inhibitor of the invention is formulated in a pharmaceutical composition, optionally comprising a pharmaceutically acceptable carrier, excipient and/or diluent.

The term "pharmaceutical composition" in accordance with the present invention relates to a composition for administration to a patient, preferably a human patient. The pharmaceutical composition of the invention comprises at least one, such as at least two, e.g. at least three, in further embodiments at least four such as at last five of the above mentioned inhibitors. The invention also envisages mixtures of inhibitors of PLD1 and inhibitors of an activator of PLD1. In cases where more than one inhibitor is comprised in the composition it is understood that none of these inhibitors has an inhibitory effect on the other inhibitors also comprised in the composition.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the bloodstream such as a brain or coronary artery or directly into the respective tissue. The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize thrombus formation or to reduce thrombus size, such as for example anti-coagulants as described in WO2006/066878, which is specifically incorporated herein in its entirety. Since the pharmaceutical preparation of the present invention relies on the above mentioned inhibitors or compounds, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The present invention further relates to a method of treating and/or preventing a disorder related to venous or arterial thrombus formation comprising administering a pharmaceutically effective amount of an inhibitor of phospholipase D1 (PLD1) or an inhibitor of an activator of PLD1 to a subject in need thereof.

The pharmaceutically effective amount of an inhibitor of phospholipase D1 (PLD1) or an inhibitor of an activator of PLD1 may be formulated as a pharmaceutical composition, as defined above.

In a preferred embodiment of the method of the invention, the subject is a mammal. More preferably, the subject is a human subject.

In a preferred embodiment of the inhibitor or the method of the invention, the inhibitor is an antibody or a fragment or derivative thereof, an aptamer, a siRNA, a shRNA, a miRNA, a ribozyme, an antisense nucleic acid molecule or a small molecule.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of PLD1 or an epitope of an activator of PLD1 (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc, are available to scientists such that the half-life of aptamers can be increased for several days or even weeks.

The term "peptide" as used herein describes a group of molecules consisting of up to 30 amino acids, whereas the term "protein" as used herein describes a group of molecules consisting of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et a/. (2008), Methods in Molecular Biology, vol, 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of PLD1 or an inhibitor of an activator of PLD1.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and *in vitro*-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays.

In a preferred embodiment of the inhibitor of the invention, the inhibitor has admixed thereto or associated in a separate container an antagonist of G-protein coupled receptors or signaling pathways. In another preferred embodiment of the inhibitor of the invention, the inhibitor has admixed thereto anti-coagulants which are known in the art, described for example in WO2006/066878, which is incorporated herein in its entirety.

In a preferred embodiment of the method of the invention, the method further comprises the administration of an antagonist of G-protein coupled receptors or signaling pathways. In another preferred embodiment of the method of the invention, the method further comprises the administration of anti-coagulants which are known in the art, described for example, in WO2006/066878 which is specifically incorporated herein in its entirety.

As used herein, the term "antagonist of G-protein coupled receptors or signaling pathways" refers to any ligand or compound that does not provoke a biological response itself upon binding to a G-protein coupled receptors, but blocks or dampens agonist-mediated responses. Thus, the antagonist has affinity but no efficacy for G-protein coupled receptors. Binding of the antagonist results in disruption of the interaction and inhibits the function of the G-protein coupled receptor. Antagonists can mediate their effects by binding to the active site or to allosteric sites on the G-protein coupled receptor, or they may interact at unique binding sites not normally involved in the biological regulation of the receptor's activity. Depending on the nature of antagonist receptor binding, the antagonist activity may be reversible or irreversible depending on the longevity of the antagonist-receptor complex.

In a more preferred embodiment of the inhibitor or the method of the invention, the antagonist of G-protein coupled receptors or signaling pathways is aspirin or is an inhibitor of P2Y1, P2Y12 or PAR receptors.

The term "aspirin", as used in accordance with the present invention, refers to acetylsalicylic acid (ASA), which is a salicylate drug. Aspirin has an antiplatelet, i.e. an "anti-coagulation", effect by inhibiting thromboxane prostaglandins, which under normal circumstances effect platelet aggregation to repair damaged blood vessels. Thus, aspirin is used in low doses in medicine for the long-term prevention of heart attacks, strokes, and blood clot formation in people at high risk for developing blood clots.

In accordance with the present invention, the term "P2Y1 receptor" refers to a seven-transmembrane receptor for adenosine diphosphate (ADP) that couples to heterotrimeric G protein (Gq) in platelets.

The term "P2Y12 receptor", according to the present invention, refers to a seven-transmembrane receptor for adenosine diphosphate (ADP) that couples to heterotrimeric G protein (Gi) in platelets.

As used herein, the term "PAR receptor" refers to seven-transmembrane protease activated receptors for thrombin (PAR1 and PAR4) that couple to heterotrimeric G proteins in platelets.

In a further preferred embodiment of the inhibitor or the method of the invention, the disorder related to venous or arterial thrombus formation is selected from the group consisting of myocardial infarction, stroke, ischemic stroke, pulmonary thromboembolism, peripheral artery disease (PAD), arterial thrombosis and venous thrombosis.

The term "myocardial infarction", as used herein, relates to a medical condition generally referred to as "heart attack" and is characterized by interrupted blood supply to the heart. The resulting ischemia (oxygen shortage) causes cellular damage and - depending on the length of ischemia - even tissue necrosis. Most commonly, myocardial infarct is due to the rupture of a vulnerable plaque that leads to a blockade of a vein or artery.

The term "stroke" is well-known in the art and is sometimes also referred to as cerebrovascular accident (CVA). A stroke is a medical condition that is medically defined by reduced blood supply to the brain resulting in loss of brain function, inter alia due to ischemia. Said reduction in blood supply can be caused, for example, by thrombosis or embolism, or due to hemorrhage. Hence, strokes are generally classified into two major categories, ie., i) ischemic and ii) hemorrhagic strokes. Ischemia is due to an interruption in blood circulation and hemorrhage is due to a rupture of a blood vessel, both scenarios ultimately leading to a reduced blood supply of the brain. The prevalent form of stroke is the ischemic stroke accounting for about 80% of strokes. In an ischemic stroke, blood supply to part of the brain is decreased, leading to dysfunction and necrosis of the brain tissue in that area. There are mainly three causative reasons: thrombosis (obstruction of a blood vessel by a blood clot forming locally), embolism (idem due to a blood clot from elsewhere in the body) and systemic hypoperfusion (general decrease in blood supply, e.g. in shock).

The term "pulmonary thromboembolism", as used in accordance with the present invention, relates to a blockage of one of the arteries in the lung by a blood clot. Microscopic thrombi, or clots, grow under certain conditions to form a larger clot that blocks a vein. This is called deep vein thrombosis (DVT). DVTs usually form in the veins of the lower leg or pelvis, but can start anywhere in the body. Pieces may break loose and travel through the bloodstream to the lung, where they block the pulmonary blood vessels and stop blood travelling through the lung to collect oxygen. This is a pulmonary embolism (PE), the result of which is dangerously low levels of oxygen in the blood and tissues.

"Peripheral artery disease (PAD)", as used herein, relates to diseases caused by the obstruction of large arteries in the arms and legs. PAD can result from atherosclerosis, inflammatory processes leading to stenosis, an embolism or thrombus formation. It causes either acute or chronic ischemia (lack of blood supply), typically of the legs.

As used in accordance with the present invention, the term "arterial thrombosis" relates to the formation of a thrombus within an artery. In most cases, arterial thrombosis follows rupture of atheroma, and is therefore referred to as atherothrombosis. There are two major diseases which can be classified under this category: stroke and myorcardial infarction.

The term "venous thrombosis", according to the present invention, relates to blood clot formation within a vein. Superficial venous thromboses can cause discomfort but generally do not cause serious consequences, unlike the deep vein thromboses (DVTs) that form in the deep veins of the legs or in the pelvic veins. Since the veins return blood to the heart, breaking off of a piece of a blood clot formed in a vein can lead to its being transported to the right side of the heart, and from there into the lungs, leading to pulmonary embolism as described above.

All of the diseases described herein are well known to the skilled person and are defined in accordance with the prior art and the common general knowledge of the skilled person.

In a more preferred embodiment of the inhibitor or the method of the invention, the stroke is ischemic stroke.

The present invention also relates to a method of identifying a compound suitable as a lead compound and/or as a medicament for the treatment and/or prevention of a disorder related to venous or arterial thrombus formation, comprising the steps of:
(a) determining the level of PLD1 protein or *pld1* transcript in thrombocytes; (b) contacting said thrombocytes or thrombocytes of the same population with a test compound; (c) determining the level of PLD1 protein or *pld1* transcript in said thrombocytes after contacting with the test compound; and (d) comparing the level of PLD1 protein or *pld1* transcript determined in step (c) with the PLD1 protein or *pld1* transcript level determined in step (a), wherein a decrease of PLD1 protein or *pld1* transcript in step (c) as compared to step (a) indicates that the test compound is a compound suitable as a lead compound and/or as a medicament for the treatment and/or prevention of a disorder related to venous or arterial thrombus formation.

This embodiment relates to a cellular screen, wherein inhibitors may be identified which exert their inhibitory activity by interfering with the expression of PLD1, either by affecting the stability of PLD1 protein or transcript (mRNA) or by interfering with the transcription or translation of PLD1.

A "compound" in accordance with the present invention can be any of the inhibitors defined above, i.e. an antibody or a fragment or derivative thereof, an aptamer, a siRNA, a shRNA, a miRNA, a ribozyme, an antisense nucleic acid molecule or a small molecule. Test compounds further include but are not limited to, for example, peptides such as soluble peptides, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids or phosphopeptides (e.g., members of random and partially degenerate, directed phosphopeptide libraries, see, e.g., Songyang et al. (1993) Cell 72: 767-778).

The term "thrombocytes or thrombocytes of the same population" as used herein refers either to the thrombocytes used in step (a) or to thrombocytes being of the same origin as the thrombocytes of step (a) and having identical characteristics to the thrombocytes of (a). Furthermore, this term both encompasses thrombocyte populations, such as for example homogenous cell populations consisting of cells having identical characteristics, as well as single thrombocytes for single cell analyses.

As described hereinabove, PLD1 is an is an essential mediator of platelet activation. Therefore, the use of PLD1 as a target for the discovery of compounds that are suitable for the treatment and/or prevention of a disorder related to venous or arterial thrombus formation is also encompassed by the present invention. It is envisaged that a decrease of expression levels of PLDlconferred by a compound as described above may contribute to protection from venous or arterial thrombus formation and may ameliorate conditions associated therewith, as described above. Accordingly, measurement of the PLD1 protein or *PLD1* transcript level may be used to determine the readout of the above-described assay.

For example, the above-mentioned thrombocytes may exhibit a detectable level of PLD1 protein or *PLD1* transcript before contacting with the test compound and the level of PLD1 protein or *PLD1* transcript may be lower or undetectable after contacting the thrombocytes with the test compound, indicating a compound suitable for the treatment and/or prevention of a disorder related to venous or arterial thrombus formation or as a lead compound for the development of a compound for this treatment. Preferably, the level of PLD1 protein or *PLD1* transcript after contacting the thrombocytes with the test compound is reduced by, for example, at least 10, 20, 30, 40 or 50% as compared to the level of PLD1 protein or *PLD1* transcript before contacting the thrombocytes with the test compound. More preferably, the level of PLD1 protein or *PLD1* transcript after contacting the thrombocytes with the test compound is reduced by, for example, at least 60, 70, 80, 90 or 95% as compared to the level of PLD1 protein or *PLD1* transcript before contacting the thrombocytes with the test compound. Most preferably, the level of PLD1 protein or *PLD1* transcript after contacting the thrombocytes with the test compound is reduced by 100% as compared to the level of PLD1 protein or *PLD1* transcript before contacting the thrombocytes with the test compound. The term "the level of PLD1 protein or *PLD1* transcript is reduced by [...] %" refers to a relative decrease compared to the level of PLD1 protein or *PLD1* transcript before contacting the thrombocytes with the test compound. For example, a reduction of at least 40% means that after contacting the thrombocytes with the test compound the remaining level of PLD1 protein or *PLD1* transcript is only 60% or less as compared to the level of PLD1 protein or *PLD1* transcript before contacting the thrombocytes with the test compound. A reduction by 100% means that no detectable level of PLD1 protein or *PLD1* transcript remains after contacting the thrombocytes with the test compound.

Measurements of protein levels as well as of transcript level can be accomplished in several ways, as described above.

The present invention further relates to a method of identifying a compound suitable as a lead compound and/or as a medicament for the treatment and/or prevention of a disorder related to venous or arterial thrombus formation, comprising the steps of:
(a) determining the level of PLD1-mediated integrin αllbβ3 activation in thrombocytes upon stimulation of the thrombocytes with an activator of PLD1; (b) contacting said thrombocytes or thrombocytes of the same population with a test compound; (c) determining the level of PLD1-mediated integrin αllbβ3 activation in thrombocytes upon stimulation of the thrombocytes with an activator of PLD1 after contacting with the test compound; and (d) comparing the level of PLD1-mediated integrin αllbβ3 activation in thrombocytes upon stimulation of the thrombocytes with an activator of PLD1 determined in step (c) with the level of PLD1-mediated integrin αllbβ3 activation in thrombocytes upon stimulation of the thrombocytes with an activator of PLD1 determined in step (a), wherein a decrease in the level of PLD1-mediated integrin αllbβ3 activation in thrombocytes in step (c) as compared to step (a) indicates that the test compound is a compound suitable as a lead compound and/or as a medicament for the treatment and/or prevention of a disorder related to venous or arterial thrombus formation.

This embodiment relates to a cellular screen, wherein inhibitors may be identified which exert their inhibitory activity by physically interacting with PLD1, or alternatively (or additionally) by functionally interacting with PLD1, i.e., by interfering with the pathway(s) present in the thrombocytes employed in the cellular assay. As a result, the biological activity of PLD1 is altered either directly or indirectly.

This method includes as a first step the stimulation of thrombocytes with an activator of PLD1 and the determination of the level of PLD1-mediated integrin αllbβ3 activation in the thrombocytes. In the next step of the method, the thrombocytes or thrombocytes of the same population are contacted with a test compound and, in a third step, the level of PLD1-mediated integrin αllbβ3 activation in the thrombocytes upon stimulation of the thrombocytes with an activator of PLD1 after contacting with the test compound is determined. The thus observed level of PLD1-mediated integrin αllbβ3 activation in the thrombocytes is compared between the first step carried out in the absence of the test compound and the third step, carried out after contacting with the test compound. If after contacting with the test compound a reduction in PLD1-mediated integrin αllbβ3 activation in the thrombocytes is observed, then the test compound is an inhibitor of PLD1-mediated integrin αllbβ3 activation and is thus suitable as a lead compound and/or as a medicament for the treatment and/or prevention of a of a disorder related to venous or arterial thrombus formation. Most preferably, the test compound results in no PLD1-mediated integrin αllbβ3 activation in the thrombocytes at all, i.e. the test compound successfully inhibits PLD1-mediated integrin αllbβ33 activation completely, i.e. to 100%.

In a preferred embodiment, the activation of integrin αllbβ3 in thrombocytes is determined by measuring the amount of thrombocyte aggregation, by determining the amount of fibrinogen binding and/or by measuring thrombocyte adhesion to von Willebrand factor under high shear flow conditions. These methods are known in the art and/or are described in detail in the examples below. The term "high shear flow conditions" as used herein refers to shear rates above 1000s⁻¹,preferably to shear rates above 1200s⁻¹ and more preferably to shear rates above 1500 s⁻¹.

In a preferred embodiment of this method of the invention, the activator of PLD1 is selected from the group consisting of GPIb-V-IX, GPVI, protease activated receptors 1 and/or 4, ADP receptors P2Y1 and P2Y12, CLEC-2 and thromboxane A2 receptor.

Protease activated receptors 1 and/or 4 (Par 1/4) and ADP receptors P2Y1 and P2Y12 have been defined elsewhere herein. In accordance with the present invention, the term "GPIb-V-IX" refers to the glycoprotein complex Ib-V-IX which constitutes the full receptor for von Willebrand factor (VWF) on platelets, while the therm "GPVI" refers to Glycoprotein VI (platelet) which is a glycoprotein receptor for collagen which is expressed in platelets. The term "CLEC-2", as used herein, relates to the "C-type lectin-like receptor 2" (GenBank database accession no. AF124841; GeneID: 51266), which is a C-type lectin-like type II transmembrane receptor that was originally identified in immune cells and only recently revealed to be expressed in platelets. The term "thromboxane A2 receptor", in accordance with the present invention, refers to a protein on the surface of cells in the endothelium of blood vessels and in the placenta which interacts with the eicosanoid lipid thromboxane. The human thrombocane A2 receptor (TXA2 receptor) is a typical G protein-coupled receptor (GPCR) with seven transmembrane segments. In humans, two TP receptor splice variants - TPa and TPβ - have so far been cloned. All of these molecules are well known to the skilled person and are defined in accordance with the prior art and the common general knowledge of the skilled person.

In a preferred embodiment, the methods of the invention are carried out *in vitro.*
*In vitro* methods offer the possibility of establishing high-throughput assays, as described above.

The identified so-called lead compounds may further be optimized to arrive at a compound which may be used in a pharmaceutical composition. Methods for the optimization of the pharmacological properties of compounds identified in screens, the lead compounds, are known in the art and comprise, for example, the methods described above for the modifications of inhibitors.

### The figures show:

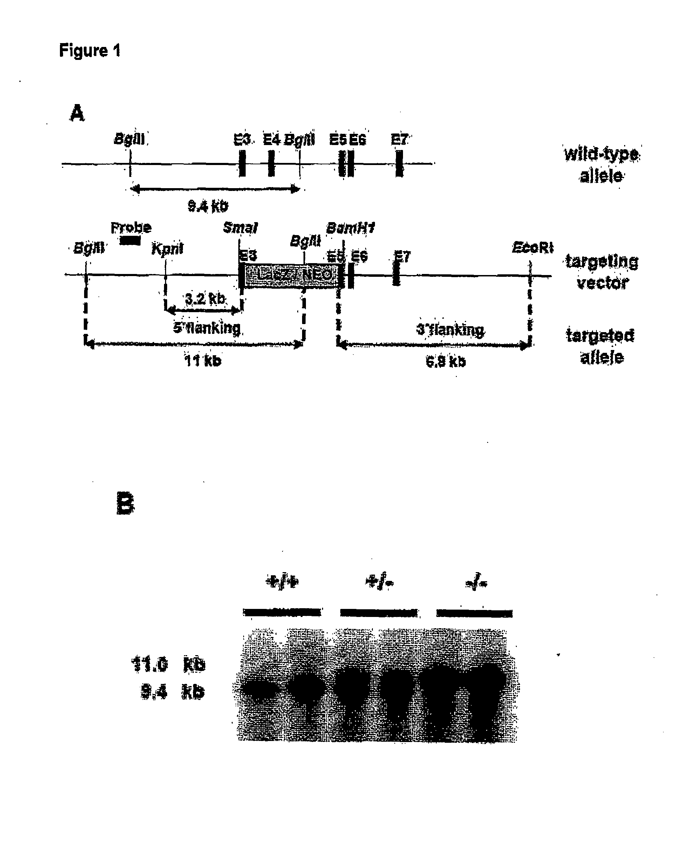

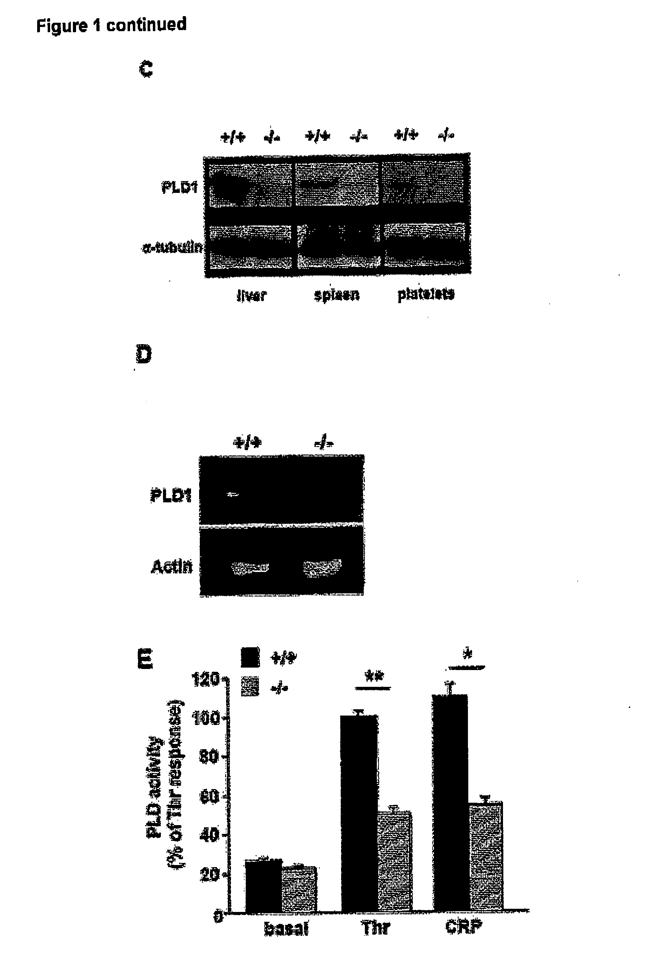

### Figure 1: Generation of PLD1-deflicient mice

**A,** Targeting strategy. Exons 3 to 5 were targeted for heterologous recombination (see Material and Methods for details); Neo-LacZ: neomycin resistance and LacZ cDNA. **B,** Southern Blot of *Bg*/II-digested genomic DNA of wild-type (+/+), heterozygous (+/-) or *Pld1*^{*-*/*-*} (-/-) mice labeled with the external probe indicated in A. **C,** Protein lysates of the indicated organs immunoblotted using anti-PLD1 (Cell Signaling) and anti-tubulin antibodies. **D,** RT-PCR of platelet cDNA from wild-type (+/+) and *Pld1*^{*-*/*-*} (-/-) platelets. **E,** PLD-activity of wild-type (+/+) and *Pld1*^{*-*/*-*} (-/-) platelets was measured (as described in Material & Methods) under resting conditions (basal) or upon stimulation with thrombin (Thr) or CRP. PLD-activity of wild-type platelets upon thrombin stimulation was set as 100%.

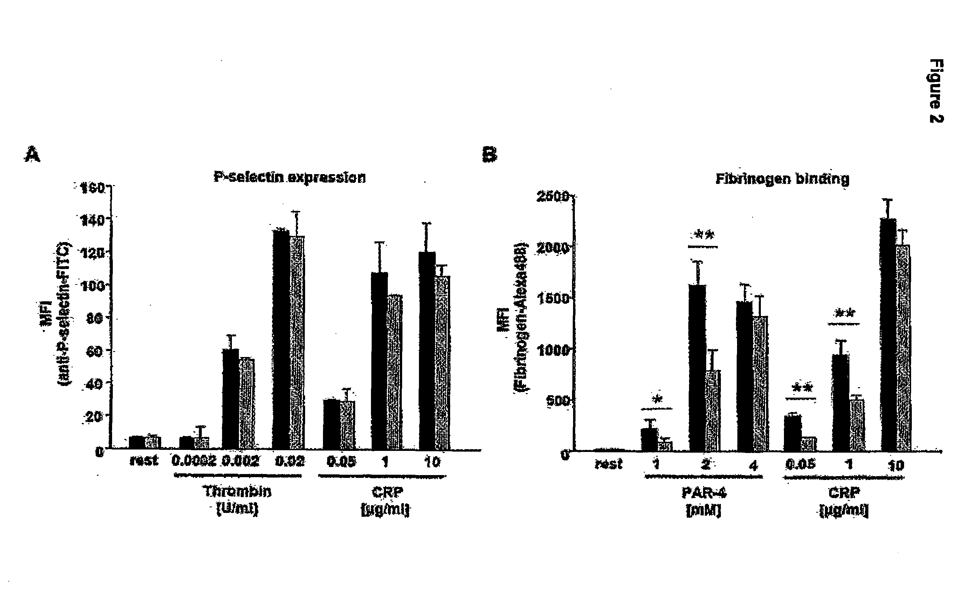

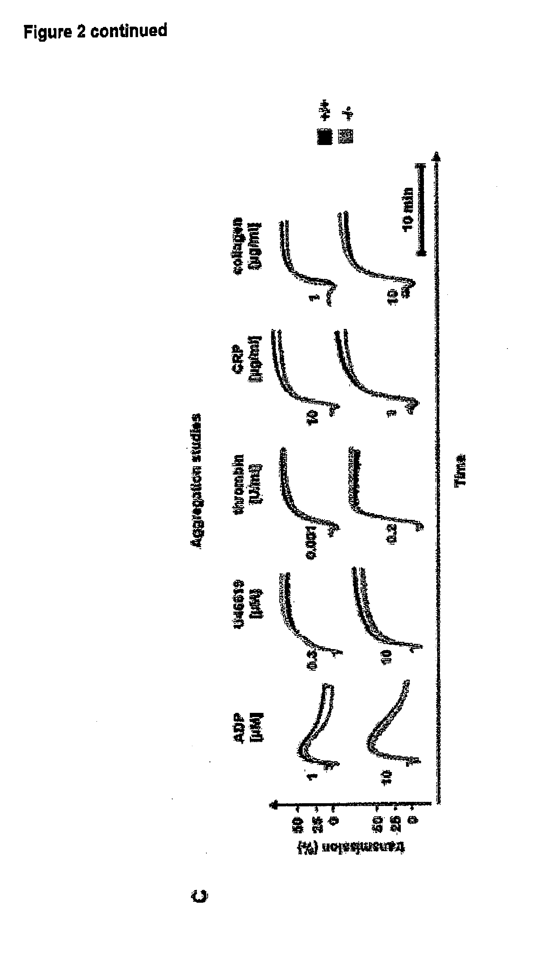

### Figure 2: Impaired integrin αllbβ3 activation in Pld1^{-/-} platelets

**A,** Washed blood from wild-type and *Pld1*^{*-*/*-*} mice was incubated for 15 min with the indicated agonists in the presence of FITC-conjugated anti-mouse P-selectin antibody. The cells were gated by FSC/SSC characteristics. Data shown are mean fluorescence intensities (MFI)±SD (n = 5 per group). B, Washed blood from wild-type and *Pld1*^{*-*/*-*} mice was stimulated with PAR4 activating peptide or CRP. The cells were gated by FSC/SSC characteristics. Data shown are mean fluorescence intensities (MFI)±SD of one representative experiment (n ≥ 4 per group). **p*<0.05; ***p*<0.01; ****p*<0.001. C, Aggregometry studies: platelets from wild-type (black line) and *Pld1*^{*-*/*-*} (gray line) mice were activated with the indicated agonists. Light transmission was recorded on a Fibrintimer 4 channel aggregometer over 10 min and is expressed as arbitrary units with 100% transmission adjusted with plasma. The results shown are representative of 3 individual experiments.

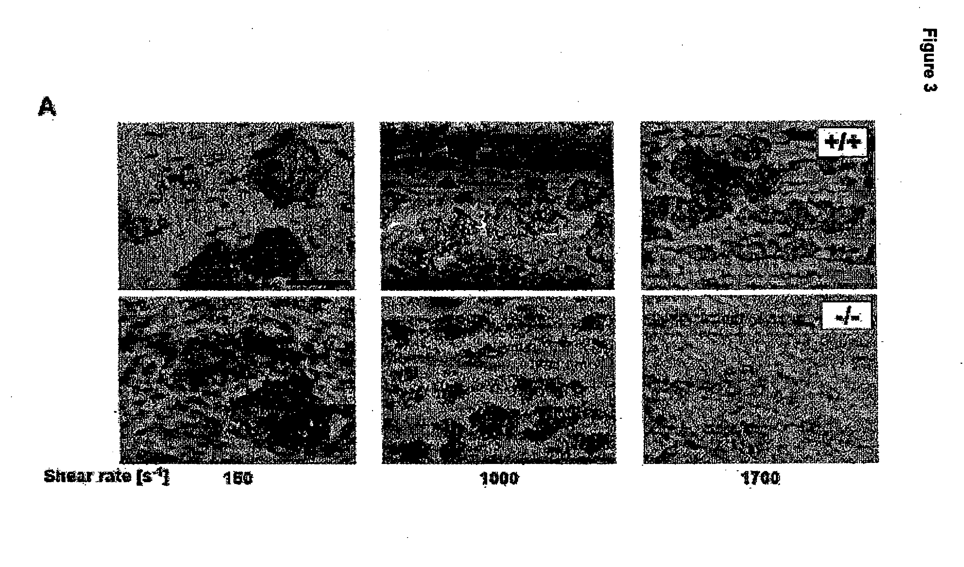

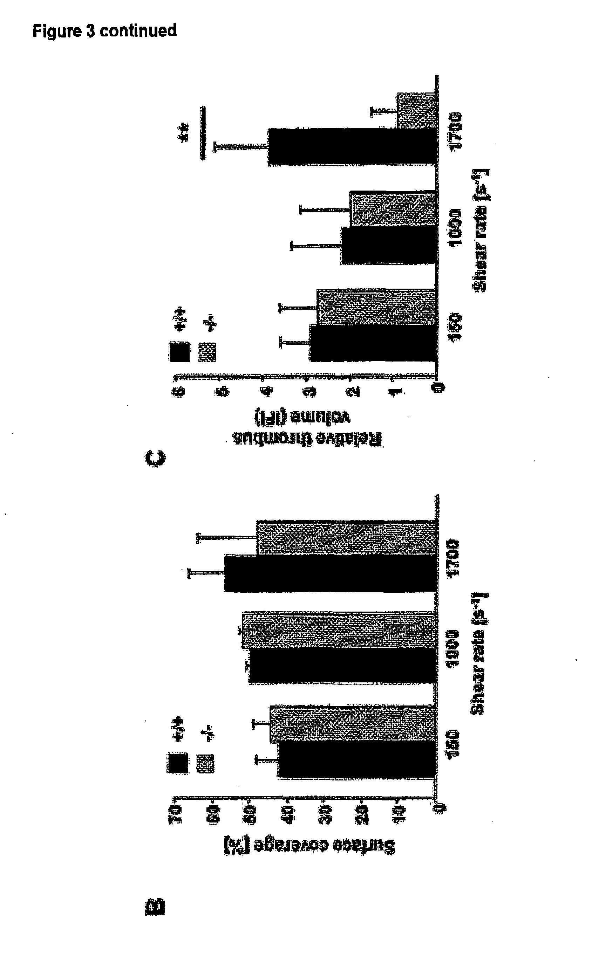

### Figure 3: Defective aggregate formation of Pld1⁻¹⁻ platelets on collagen at high shear

Whole blood was perfused over a collagen-coated (0.2 mg/ml) surface with the indicated shear rates, and then washed with Tyrode's buffer for the same perfusion time. **A,** Representative phase-contrast images at a shear rate of 1700 s⁻¹. Bar, 100 µm **B,** Mean surface coverage (left) and C, relative platelet deposition as measured by the integrated fluorescent intensity per visual field (right). All bar graphs depict mean values ± SD (n ≥ 3 mice each). **p*<0.05; ***p*<0.01; ****p*<0,001.

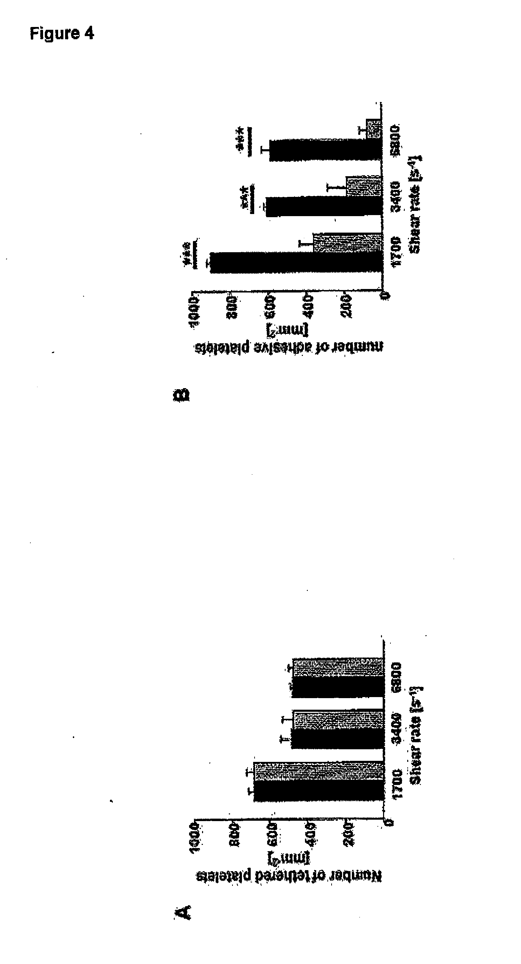

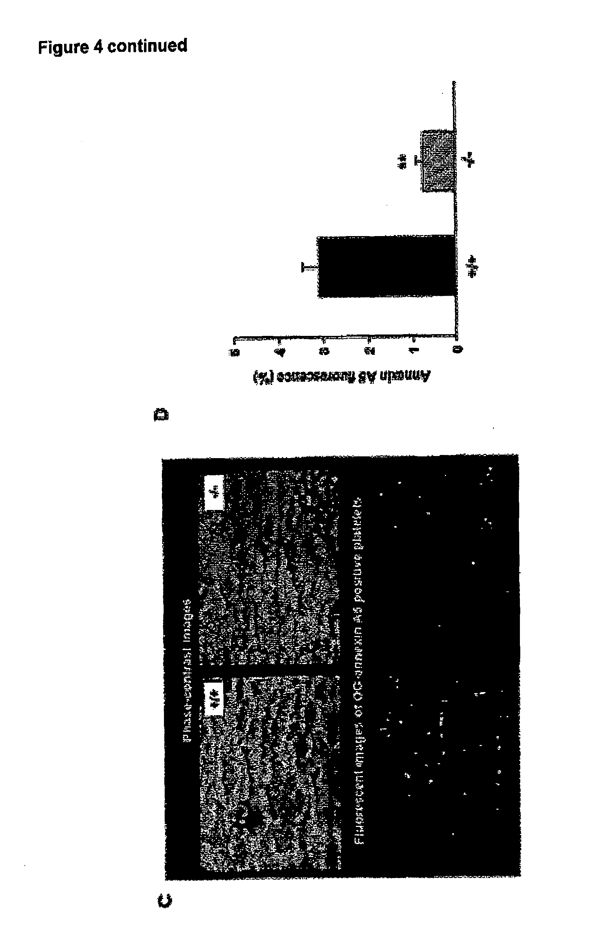

### Figure 4: Pld1^{-/-} platelets fail to firmly adhere to vWF under flow and display reduced coagulant activity

Whole blood was perfused over a collagen-coated or vWF-coated (indirect coating using anti-vWF and mouse plasma; see Material & Methods) surface with the indicated shear rates, and then washed with Tyrode's buffer for the same perfusion time. **A, B,** Adhesive platelets on the vWF-coated surface were counted after 100 s of blood perfusion **(A)** or after the end of the 4 min blood perfusion and washing step, reflecting firmly adhered platelets **(B)**. All bar graphs depict mean values ± SD (n ≥ 3 mice each). **p*<0.05; ***p*<0.01; ****p*<0.001. **C-D,** Whole blood was perfused over a collagen-coated (0.2 mg/ml) surface at a shear rate of 1,000 s⁻¹ for 4 min. After the run the adhered platelets were stained with 0.5 µg/ml OG-annexin A5. **C,** Representative phase-contrast (top) and fluorescence images (bottom). Scale bar 100 µm. **D,** Mean relative amount of annexin A5 positive platelets ± SD (n ≥ 3 mice). **p*<0.05; ***p*<0.01; ****p*<0.001.

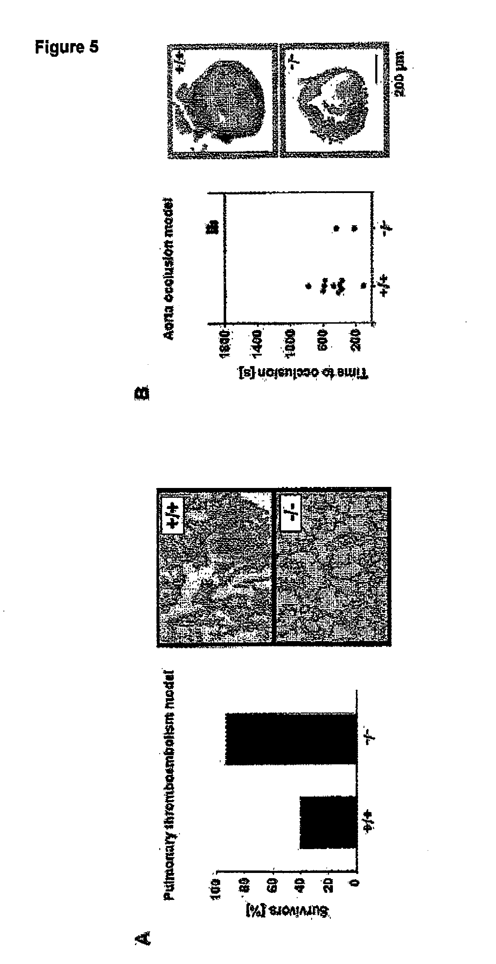

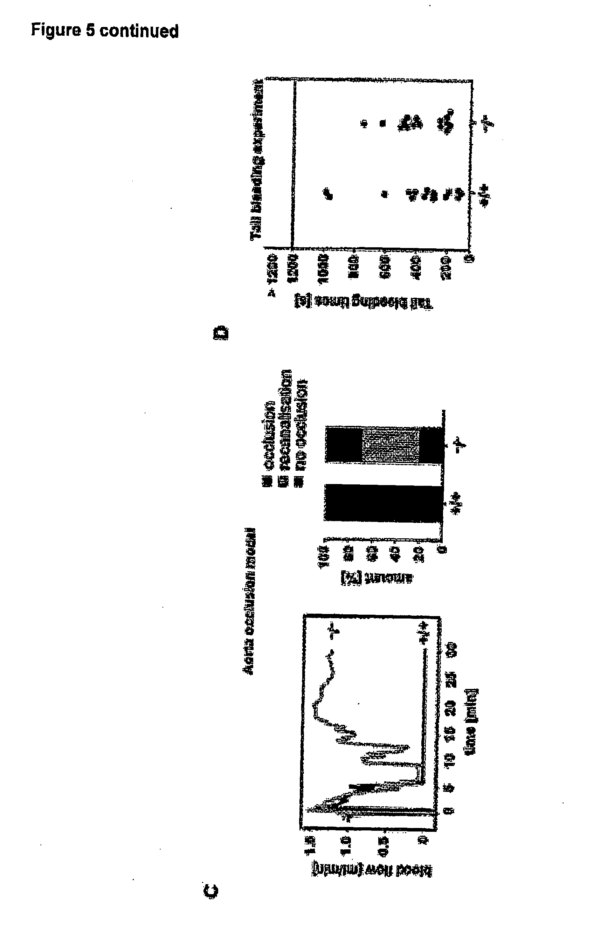

### Figure 5: Reduced thrombus stability of Pld1^{-/-} platelets in vivo

**A,** Lethal pulmonary embolization after injection of collagen and epinephrine in anesthetized wildtype (+/+) and *Pld1*^{*-*/*-*} (-/-) mice. The left panel displays the survivor rate upon injection of 700 µg collagen and 60 µg epinephrine per kg bodyweight and the right panel shows representative pictures of lung sections. B, **C,** Mechanical injury of the abdominal aorta of wild-type (+/+) and *Pld1*^{*-*/*-*} (-/-) mice was performed and blood flow was monitored with a Doppler flowmeter. **B,** Time to final occlusion, each symbol represents one individual and representative cross-sections of the aorta 30 min after injury are shown. Bars represent 200 µm. **C,** Representative time course of blood flow in wild-type (black line) or *Pld1*^{*-*/*-*} (grey line) aortas and percentage distribution of irreversible occlusion (black), unstable occlusion (light grey) and no occlusion (dark gray). **D,** Tail bleeding times in wild-type and *Pld1*^{*-*/*-*} mice. Each symbol represents one animal.

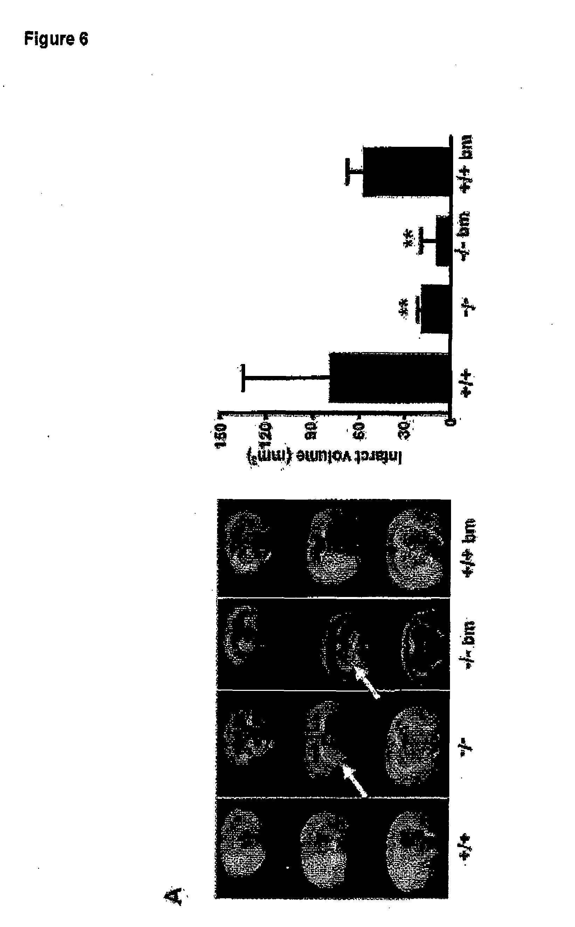

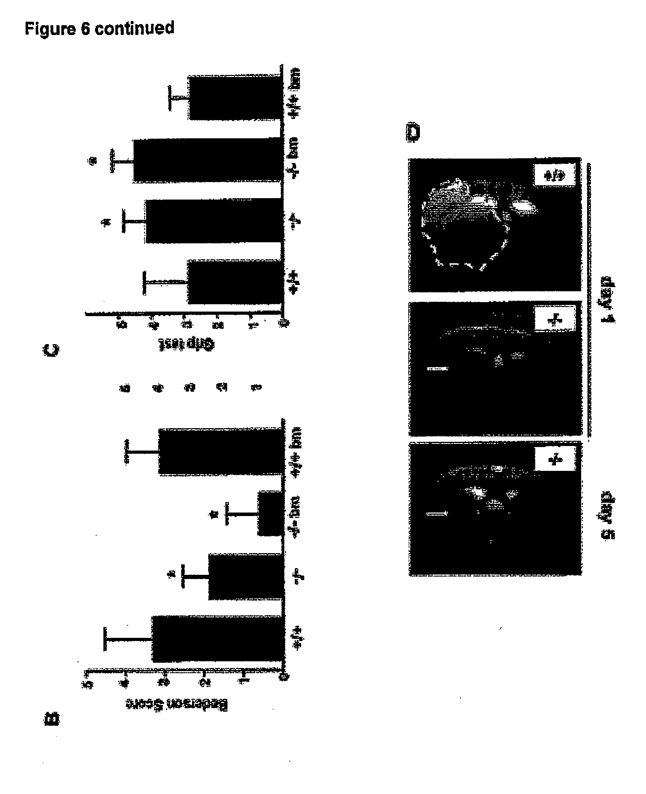

### Figure 6: Pld1^{-/-} mice are protected from cerebral ischemia

**A,** Representative images of three corresponding coronal sections from control (+/+), *Pld1*^{*-*/*-*} (-/-), wild-type mice transplanted with *Pldl^{-l-}* bone marrow (-/- bm) and *Pld1*^{*-*/*-*} mice transplanted with wild-type bone marrow (+/+ bm) stained with TTC 24 h after tMCAO. Right panel: brain infarct volumes of these mice. **B, C,** Neurological Bederson score and grip test assessed at day 1 following tMACO of the mice mentioned in A. **D,** The coronal T2-w MR brain image shows a large hyperintense ischemic lesion at day 1 after tMCAO in controls (+/+). Infarcts are smaller in *Pld1*^{*-*/*-*} mice (-/-, white arrow), and T2 hyperintensity decreases by day 5 during infarct maturation (-/-; lower panel). Importantly, hypointense areas indicating intracerebral hemorrhage were not seen in *Pld1*^{*-*/*-*} mice, demonstrating that PLD1 deficiency does not increase the risk of hemorrhagic transformation, even at advanced stages of infarct development. Data are mean ± SD (n = 10 per group). * *p*<0.05; ** *p*<0.01; *** *p*<0.001.

The examples illustrate the invention.

### Example 1: Materials and methods

**Animals.** Animal studies were approved by the district government of Lower Franconia (Bezirksregierung Unterfranken). The generation of *P*/*d1*^{*-*/*-*} mice was as follows. The murine *Pld1* gene (108 kb) was identified in the P1 artifical chromosome (PAC) mouse library (RPCI22) (HGMP Resource Centre, UK) with PLD1 specific probe and ordered from CHORI. Intron2-exon3 specific Kpnl-Smal genomic fragment (3.2 kb) and exon5-intron7 specific BamHI-EcoRI genomic fragment (6.8 kb) from PLD1 PAC genomic clone were subcloned into pBluescript/SK, sequenced and inserted into a pWH9 targeting vector consisting of an internal ribosomal entry site (IRES), a LacZ reporter gene, and a neomycin-resistant cassette with PGK promoter and poly(A) signal sequences. These genomic fragments were used as 5'- and 3'- homologous arm, respectively. PLD1 targeting vector was linearized and electroporated into R1 embryonic stem (SV129) cells.

After C418 selection, 400 resistant ES cell colonies were isolated and screened for homologous recombination by probing BgIII digested genomic ES cell DNA with PLD1 specific external probe. Targeted ES cell clones were injected into blastocysts of pseudo-pregnant C57BL/6 females to generate germ line chimeras. Male chimeras were bred to C57BL/6 females to generate *Pld1*^{+/-} mice, which were intercrossed to produce *Pld1*^{-/-} mice and littermate wild-type controls. Genotyping was performed with Southern blotting to detect wild-type and targeted allele of PLD1.

**Chemicals and antibodies.** Anesthetic drugs used included medetomidine (Pfizer), midazolam (Roche), and fentanyl (Janssen-Cilag). To undo anesthesia, atipamezol (Pfizer) and flumazenil and naloxon (both purchased from Delta Select) were used according to local authority regulations. ADP (Sigma-Aldrich), U46619 (Qbiogene), thrombin (Roche), collagen (Kollagenreagent Horm; Nycomed) were purchased as indicated. Monoclonal antibodies conjugated to FITC, PE or DyLight 488 were obtained from Emfret Analytics. The JON/A-PE antibody preferentially binds to the high affinity conformation of mouse integrin αllbβ3 (Emfret Analytics). Anti-PLD1 monoclonal antibodies were purchased from Cell Signaling.

**PLD activity measurements.** In a nonradioactive method PLD-mediated PA production was analyzed using a fluorescent *in vitro* assay. In this enzymatically coupled assay phospholipase D hydrolyses PC in the presence of PIP₂ to PA and choline, which is then oxidized by choline oxidase to betaine and H₂O₂. In the presence of HRP, H₂O₂ oxidizes Amplex red in a 1:1 stoichiometry to generate fluorescent resorufin (7-hydroxy-3H-phenoxazin-3-one). This fluorescent assay effectively screens PLD activity in the presence and absence of activators. PLD activity is expressed as a percentage of that obtained with thrombin.

Washed platelets were adjusted to a concentration of 1×10⁶/µl. Platelets were activated with the indicated agonists for 30 min at 37°C under stirring conditions (350 rpm). Platelets were lysed subsequently. Samples were collected and 100 µl of the samples were mixed with 100 µl of the Amplex red reaction buffer (Amplex^{®}Red phospholipase D assay kit, Molecular Probes). The PLD activity was determined by duplicate for each sample by measuring fluorescence activity after 1-h incubation at 37°C in the dark with the FluorScan. A standard curve was performed with purified PLD from Streptomyces chromofuscus (Sigma-Aldrich) with concentrations ranged from 0-250 mU/ml.

**Platelet aggregation and flow cytometry.** Washed platelets (200 µl with 0.5×10⁶ platelets/µl) were analyzed in the presence of 70 µg/ml human fibrinogen. Transmission was recorded on a four-channel aggregometer (Fibrintimer; APACT) for 10 min and was expressed in arbitrary units, with buffer representing 100% transmission. For flow cytometry, heparinised whole blood was diluted 1:20, and incubated with the appropriate fluorophore-conjugated monoclonal antibodies for 15 min at room temperature and analyzed on a FACSCalibur (Becton Dickinson).

**Fibrinogen binding**. Washed platelets were incubated with 50 µg/ml human Alexa-488 labelled fibrinogen for 10 min and activated with the respective agonist for 20 min at room temperature. Analysis was done on a FACSCalibur (Becton Dickinson).

**Adhesion under flow conditions.** Rectangular coverslips (24 × 60 mm) were coated with 0.2 mg/ml fibrillar type I collagen (Nycomed) or rabbit anti-human vWF antibody (1:500; DakoCytomation, A0082) for 1 h at 37°C and blocked with 1% BSA. For flow adhesion experiments on vWF, blocked coverslips were incubated with murine plasma for 2 h at 37°C to allow binding of vWF to immobilized anti-vWF antibody. Heparinized whole blood was labeled with a DyLight 488-conjugated anti-GPIX Ig derivative at 0.2 µg/ml, and perfusion was performed as previously described (33). Image analysis was performed off-line using Metavue software (Visitron). Thrombus formation was expressed as the mean percentage of total area covered by thrombi, and as the mean integrated fluorescence intensity per square millimeter. Platelet adhesion on the vWF matrix was analyzed by counting adhesive platelets per visual field.

**Determination of phosphatidylserine-exposing platelets after perfusion.** Adhesion experiments under flow conditions (1000 s⁻¹) were performed with heparanized whole blood containing PPACK(34). Rectangle coverslips were coated with type I collagen (Nycomed), rinsed with saline and blocked with 1% BSA. To prevent coagulation, chamber and tubing were prewashed with HEPES buffer supplemented with 1 U/ml heparin. The blood was perfused through the flow chamber by using a 1-ml syringe and a pulse-free pump at a shear rate of 1000 s⁻¹ for 4 min. The flow chamber was perfused with HEPES buffer supplemented with 1 U/ml heparin at the same shear rate for 10 min. Exposure of PS was detected with OG488-labeled annexin A5 (1 µg/ml). Phase-contrast and fluorescent images were obtained from at least seven different collagen-containing microscopic fields, which were randomly chosen.

**Bleeding time**. Mice were anesthetized and a 3-mm segment of the tail tip was removed with a scalpel. Tail bleeding was monitored by gently absorbing blood with filter paper at 20 s intervals without making contact with the wound site. When no blood was observed on the paper, bleeding was determined to have ceased. Experiments were stopped after 20 min.

**Pulmonary thromboembolism model.** Mice were anesthetized by intraperitoneal injection of triple anesthesia, 0.15 ml/10 g body weight from 2.5% solution. Anesthetized mice received a mixture of collagen (0.7 mg/kg) and epinephrine (60 µg/kg) injected into the jugular vein. The incisions of surviving mice were stitched, and the mice were allowed to recover.

**Aorta occlusion model.** A longitudinal incision was used to open the abdominal cavity and expose the abdominal aorta of anesthetized mice. An ultrasonic flow probe was placed around the vessel and thrombosis was induced by a single firm compression with a forceps. Blood flow was monitored for 30 min.

**tMCA occlusion model.** Experiments were conducted on 10-12 weeks-old *Pld1*^{*-*/*-*} and control mice or corresponding chimeras according to previously published recommendations for research in mechanism-driven basic stroke studies(35). tMCAO was induced under inhalation anesthesia using the intraluminal filament (Doccol Company) technique (22). After 60 min, the filament was withdrawn to allow reperfusion. For measurements of ischemic brain volume, animals were killed 24 h after induction of tMCAO, and brain sections were stained with 2% TTC (Sigma-Aldrich). Brain infarct volumes were calculated and corrected for edema (22). Neurological function and motor function were assessed by two independent and blinded investigators 24 h after tMACO, as previously described (22).

**Assessment of infarction and hemorrhage by MRI.** MRI was performed 24 h and 7 d after transient ischemia on a 1.5 T unit (Vision; Siemens) under inhalation anesthesia. A custom-made dual-channel surface coil was used for all measurements (A063HACG; Rapid Biomedical). The MR protocol included a coronal T2-w sequence (slice thickness = 2 mm) and a coronal T2-w gradient echo constructed interference in steady state (CISS) sequence (slice thickness = 1 mm). MR images were transferred to an external workstation (Leonardo; Siemens) for data processing. The visual analysis of infarct morphology and the search for eventual intracerebral hemorrhage were performed in a blinded manner. Infarct volumes were calculated by planimetry of hyperintense areas on high resolution CISS images.

**Histology.** Formalin-fixed brains embedded in paraffin (HistoLab) were cut into 4-µm thick sections and mounted. After removal of paraffin, tissues were stained with hematoxylin and eosin (Sigma-Aldrich).

**Statistics.** Results from at least three experiments per group are presented as means ± SD. Differences between wild-type and *Pld1*^{*-*/*-*} groups were assessed by the Mann Whitney U test. For the stroke model, results are presented as means ± SD. Infarct volumes and functional data were tested for Gaussian distribution with the D'Agostino and Pearson omnibus normality test and then analyzed using the two-tailed Student's *t* test. For statistical analysis, PrismGraph 4.0 software was used, *p*<0.05 was considered statistically significant.

### Example 2: Generation of a PLD1-deficient mouse line

To analyze the role of PLD1 *in vivo* the *Pld1* gene was disrupted in mice (Fig. 1A, B). Mice heterozygous for the *Pld1*-null mutation as well as PLD1-deficient (*Pld1*^{*-*/*-*}) mice were born in expected Mendelian ratio, developed normally, were fertile and showed normal behavior. The animals appeared healthy and did not exhibit spontaneous bleeding. Western blot analysis (Fig. 1C) and RT-PCR (Fig. 1D) confirmed the absence of PLD1 in platelets and other tissues. Blood platelet counts were normal, but the mean platelet volume was slightly increased compared to the wild-type, which was also associated with slightly increased surface expression levels of most of the major platelet surface receptors, including glycoprotein (GP) V, CD9, β1 and β3 integrins. No differences were found in red and white blood cell counts and hematocrit (Table 1). Platelets from PLD1-deficient mice had a normal phospholipid composition (data not shown). These results demonstrated that PLD1 is not essential for megakaryopoiesis and platelet production.

| | wt | *Pld1*^{*-*/*-*} |
|---|---|---|
| **platelets** | 849 ± 257 | 889 ± 322 |
| **MPV (fl)** | 5.13 ± 0.25 | 5.58 ± 0.18 |
| **Red blood cells** | 9.53 ± 1.53 | 9.14 ± 1.88 |
| **White blood cells** | 7.98 ± 2.76 | 6.93 ± 1.99 |
| **HCT [%]** | 48.93 ± 7.54 | 45.78 ± 9.28 |
| **GPIb** | 367 ± 7 | 373 ± 30 |
| **GPV** | 335 ± 7 | 358 ± 9 |
| **GPIX** | 521 ± 16 | 542 ± 22 |
| **GPVI** | 40 ± 4 | 49 ± 8 |
| α**2** | 99 ± 5 | 105 ± 2 |
| β**1** | 144 ± 3 | 176 ± 2 |
| α**IIb**ß**3** | 658 ± 24 | 691 ± 25 |
| **CD9** | 1491 ± 109 | 1710 ± 54 |

**Table 1: Blood cell counts and platelet glycoprotein expression of *Pld1*^{*-*/*-*}mice.** Hematology, hemostasis and glycoprotein expression in *Pud1*^{*-*/*-*} mice. Platelet and erythrocyte counts per nl, hematocrit and glycoprotein expression in *Pld1*^{*-*/*-*} mice. To analyze glycoprotein-expression diluted whole blood was stained with fluorophore-labeled antibodies at saturating concentrations for 15 min at room temperature and measured by flow cytometry using the FACScalibur (Becton Dickinson, Heidelberg). Results are given as the mean fluorescence intensity ± SD of at least 9 mice per group. The abbreviations are: mean platelet volume (MPV) and hematocrit (HCT).

### Example 3: Impaired integrin αIIbβ3 activation in Pld1^{-/-} platelets

Basal PLD activity was comparable between wild-type and *Pld1*^{*-*/*-*} platelets (Fig. 1 E), suggesting that it derives largely from activity of the PLD2 isoform. However, while thrombin and the GPVI-stimulating *collagen-related peptide* (CRP) induced a marked increase in PLD activity in wild-type platelets, the response in *Pld1*^{*-*/*-*} platelets was substantially blunted. These results indicated that PLD1 is responsible for the bulk of induced PLD activity in stimulated platelets.

To analyze the functional consequences of PLD1 deficiency, platelet activation in response to PAR4-activating peptide and CRP was assessed by flow cytometric analysis of P-selectin exposure as a reporter for degranulation, and by binding to Alexa-Fluor 488-tagged fibrinogen as a reporter for integrin αIIbβ3 activation. While agonist-induced degranulation was not affected in *PId1*^{*-*/*-*} platelets (Fig. 2A), agonist-induced binding of fibrinogen was strongly reduced in response to submaximal doses of PAR-4 peptide and CRP (Fig. 2B). However, at high agonist stimulatory concentrations, the defect was overcome (Fig. 2B). These results demonstrate that PLD1 is required for full integrin αIIβ3 activation downstream of the major signaling pathways in platelets in the setting of submaximal stimulation. However, this defect did not result in altered responses to major agonists such as ADP, collagen or thrombin in standard aggregometry, even at threshold agonist concentrations (Fig. 2C), confirming that robust aggregation can be observed in this setting under conditions of submaximal integrin αIIbβ3 activation²⁵.

### Example 4: PLD1 is required for stable aggregate formation under high shear flow conditions

To test the functional consequences of the altered integrin activation under more physiological conditions, platelet adhesion and thrombus formation on a collagen-coated surface was analyzed under flow in a whole blood perfusion system. Under low and intermediate shear conditions (150, 1000 s⁻¹), no significant differences in adhesion and stable aggregate formation were detected between wild-type and *Pld1*^{*-*/*-*} blood (Fig. 3A-C). Under high shear conditions (1700 s⁻¹), however, *Pld1*^{*-*/*-*}platelets displayed a severe defect in stable aggregate formation. Both wild-type and mutant platelets adhered to the collagen matrix and initiated the formation of aggregates, but in contrast to the wild-type, aggregates in *Pld1*^{*-*/*-*} blood were consistently unstable and washed away. As a result, virtually no aggregates were observed after rinsing the chamber; instead, the surface was covered mostly by a single platelet layer (Fig. 3A, C). These findings suggested that the formation and stabilization of platelet thrombi requires PLD1 under high shear flow conditions, but not under low shear conditions.

### Example 5: PLD1 is essential for efficient GPIb-dependent αIIbβ3 activation

To further investigate the shear-dependent thrombus instability in the absence of PLD1, platelet adhesion to immobilized vWF under flow was assessed. Under high shear conditions, this process involves the initial tethering of the cells through GPlb-vWF interactions, followed by the activation of integrin αIIbβ3 and transition to firm adhesion, but the underlying intracellular signaling pathways are not fully understood^{5;6}. To test a possible role of PLD1 in this process, wild-type and *Pld1*^{*-*/*-*} blood was perfused over immobilized murine vWF at different shear rates. Under all shear rates tested, the vast majority of wild-type platelets that initially attached to the vWF matrix underwent stable adhesion (Fig. 4A). In sharp contrast, while the initial tethering of *Pld1*^{*-*/*-*} platelets was indistinguishable from the wild-type control (Fig. 4A), the transition to firm adhesion was severely impaired (Fig. 4B), resulting in a strongly reduced number of adherent cells at the end of the experiment. This defect was most prominent at very high shear rates (3400 s⁻¹, 6800 s⁻¹) where adhesion is entirely GPlb-dependent. These data suggested that the absence of PLD1 affected GPlb-dependent integrin αIIbβ3 activation and stable binding to vWF under flow.

Activated integrin αIIbβ3 has been implicated in the coagulant activity of platelets²⁶. To determine a possible involvement of PLD1 in this process, whole blood from wild-type and *Pld1*^{*-*/*-*} mice was anticoagulated with PPACK and heparin, thereby maintaining physiological Ca²⁺ and Mg²⁺ concentrations, and perfused over fibrillar collagen at a wall shear rate of 1000s⁻¹, where GPlb-vWF interaction is known to contribute to collagen-induced procoagulant activity²⁷. During and after perfusion, fluorescence microscopic images were taken, In line with the previous experiments, surface coverage was unaltered (data not shown) but annexin A5 staining, reflecting phosphatidylserine exposure, was markedly reduced in *Pld1*^{*-*/*-*} platelets (Fig. 4C-D), representing a reduced proportion of procoagulant platelets.

### Example 6: PLD1-deficient mice are protected from pathological thrombus formation

To assess the function of PLD1 in platelet activation *in vivo,* wild-type and *Pld1*^{*-*/*-*} mice were intravenously injected with collagen/epinephrine which causes lethal pulmonary thromboembolism. While only 40% of the wild-type mice survived the challenge, 93% of *PId1*^{*-*/*-*} mice survived and underwent complete recovery (Fig. 5A). To investigate the significance of PLD1 for shear-dependent arterial thrombus formation *in vivo,* thrombosis was induced mechanically in the aorta by firm compression with a forceps and blood flow monitored with an ultrasonic flow probe (Fig. 5B, C). After a transient increase directly after injury (mins 0-1), blood flow progressively decreased for several minutes in all animals (mins 5-10 in the examples shown, Fig. 5C). In all wild-type mice examined, the decrease progressed to complete and irreversible occlusion of the vessel 1.6 to 12.4 min after injury (mean occlusion time: 7.4 ± 2.9 min). In sharp contrast, only 20% of the *Pld1*^{*-*/*-*} mice occluded irreversibly within the observation period (30 min) (Fig. 5B, C). In 30% of *Pld1*^{*-*/*-*} mice, no occlusion occurred, whereas in 50% of the animals the vessels occluded for <1 min, then recanalized and remained open for the remainder of the observation period (Fig. 5C). These results demonstrate that PLD1 is required for occlusive arterial thrombus formation *in vivo.*

To test whether the defective thrombus stability in *PId1*^{*-*/*-*} mice impaired hemostasis, tail bleeding experiments were performed. No significant differences in bleeding times were found between wild-type and *Pld1*^{*-*/*-*} mice (6.1 ± 4.0 min vs 5.6 ± 3.0 min, p>0.05; Fig. 5D), showing that PLD1 is not required for normal hemostasis.

### Example 7: Pld1^{-/-} mice are resistant to neuronal damage following focal cerebral ischemia

The observed thrombus instability in *Pld1*^{*-*/*-*} mice provided a strong rationale to address the importance of PLD1 in a model of focal cerebral ischemia where infarct development is to a great extent dependent on GPlb-vWF-mediated platelet adhesion and activation^{28;29}. Therefore, the development of neuronal damage in *Pld1*^{*-*/*-*} mice following transient middle cerebral artery (tMCA) occlusion was studied. A thread was advanced through the carotid artery into the MCA, reducing regional cerebral flow by >90% to induce cerebral ischemia, and removed after 1 h to allow reperfusion²⁸. In *Pld1*^{*-*/*-*} mice, infarct volumes 24 h after reperfusion, as assessed by 2,3,5-triphenyltetrazolium chloride (TTC) staining, were reduced by 80% in comparison to the infarct volumes of wild-type mice (wt: 89.9 ± 43.5 mm³; *Pld1*^{*-*/*-*}: 18.03 ± 3.43 mm³; *p*<0.01) (Fig. 6A). Reduction of infarct size was functionally relevant, since the Bederson Score assessing global neurological function (wt: 3.29 ± 1.25; *Pld1*^{*-*/*-*}: 1.86 ± 0.69; *p*<0.05) and the grip test (wt: 2.86 ± 1.35; *Pld1*^{*-*/*-*}: 4.14 ± 0.69; *p*<0.05), which specifically measures motor function and coordination, were significantly better in *Pld1*^{*-*/*-*} mice compared to wild-type controls (Fig. 6B, C). Serial magnetic resonance imaging (MRI) on living mice confirmed the protective effect of PLD1 deficiency on infarct development (Fig. 6D). Infarct volume did not increase between day 1 and day 5, suggesting a sustained protective effect of PLD1 deficiency. Furthermore no intracranial hemorrhage was detected (Fig. 6D), indicating that PLD1 deficiency is not associated with an increase in bleeding complications in the brain. Importantly, when *Pld1*^{*-*/*-*} mice were reconstituted with wild-type bone marrow ("+/+ bm", Fig. 6A-C), they developed regular infarcts, whereas only small infarctions were found in wild-type mice transplanted with *Pld1*^{*-*/*-*} bone marrow ("-/- bm"). These results show that the protection of *Pld1*^{*-*/*-*} mice from lesion formation in this model ensues from the lack of PLD1 in the hematopoietic system.

### References

1. Z. M. Ruggeri, Nat.Med. 8, 1227-1234 (2002).
2. D. L. Bhatt and E. J. Topol, Nat.Rev.Drug Discov. 2, 15-28 (2003).
3. G. Stoll, C. Kleinschnitz, B. Nieswandt, Blood 112, 3555-3562 (2008).
4. B. Savage, F. mus-Jacobs, Z. M. Ruggeri, Cell 94, 657-666 (1998).
5. Y. Ozaki, N. Asazuma, K. Suzuki-Inoue, M. C. Berndt, J. Thromb.Haemost 3, 1745-1751 (2005).
6. Z. M. Ruggeri and G. L. Mendolicchio, Circ.Res. 100, 1673-1685 (2007).
7. U. J. Sachs and B. Nieswandt, Circ.Res. 100, 979-991 (2007).
8. W. S. Nesbitt et al., Nat.Med. (2009).
9. S. Offermanns, C. F. Toombs, Y. H. Hu, M. I. Simon, Nature 389, 183-186 (1997).
10. J. R. Crittenden et al., Nat.Med. 10, 982-986 (2004).
11. M. Chrzanowska-Wodnicka, S. S. Smyth, S. M. Schoenwaelder, T. H. Fischer, G. C. White, J.Clin.Invest 115, 680-687 (2005).
12. D. A. Calderwood, J.Cell Sci. 117, 657-666 (2004).
13. B. G. Petrich et al., J.Exp.Med. 204, 3103-3111 (2007).
14. M. Moser, B. Nieswandt, S. Ussar, M. Pozgajova, R. Fassler, Nat.Med. 14, 325-330 (2008).
15. B. Nieswandt et al., J.Exp.Med. 204, 3113-3118 (2007).
16. V. Martel et al., J.Biol.Chem. 276, 21217-21227 (2001).
17. P. Huang and M. A. Frohman, Expert.Opin.Ther.Targets. 11, 707-716 (2007).
18. M. McDermott, M. J. Wakelam, A. J. Morris, Biochem.Cell Biol. 82, 225-253 (2004).
19. A. Honda et al., Cell 99, 521-532 (1999).
20. A. Nishikimi et al., Science 324, 384-387 (2009).
21. S. M. Knoepp et al., Mol.Pharmacol. 74, 574-584 (2008).
22. C. Kleinschnitz et al., Circulation 115, 2323 (2007).
23. A. Papathanasiou et al., Hellenic J Gardiol 48: 352-363 (2007).
24. M. Vorland and H. Holmsen, Platelets. 19, 211-224 (2008).
25. B. Nieswandt, V. Schulte, A. Zywietz, M. P. Gratacap, S. Offermanns, J. Biol. Chem. 277, 39493-39498 (2002).
26. J. C. Reverter et al., J.Clin.Invest 98, 863-874 (1996).
27. M. J. Kuijpers et al., J.Physiol 558, 403-415 (2004).
28. C. Kleinschnitz et al., Circulation 115, 2323-2330 (2007).
29. C. Kleinschnitz et al., Blood 113, 3600-3603 (2009).
30. H. P. Adams, Jr. et al., Stroke 39, 87-99 (2008).
31. C. J. Murray and A. D. Lopez, Lancet 349, 1269-1276 (1997).
32. R. P. Rodgers and J. Levin, Semin.Thromb.Hemost. 16, 1-20 (1990).
33. B. Nieswandt et al., EMBO J. 20, 2120 (2001).
34. J. W. Heemskerk, M. A. Feijge, E. Rietman, G. Hornstra, FEBS Lett. 284, 223 (1991).
35. U. Dimagl, J. Cereb. Blood Flow Metab 26, 1465 (2006).

## Claims

1. An inhibitor of phospholipase D1 (PLD1) or an inhibitor of an activator of PLD1 for use in treating or preventing a disorder related to venous or arterial thrombus formation or as a lead compound for developing a drug for treating or preventing a disorder related to venous or arterial thrombus formation.

2. A method of treating and/or preventing a disorder related to venous or arterial thrombus formation comprising administering a pharmaceutically effective amount of an inhibitor of phospholipase D1 (PLD1) or an inhibitor of an activator of PLD1 to a subject in need thereof.

3. The inhibitor of claim 1 or the method of claim 2, wherein the inhibitor is an antibody or a fragment or derivative thereof, an aptamer, a siRNA, a shRNA, a miRNA, a ribozyme, an antisense nucleic acid molecule or a small molecule.

4. The inhibitor of any one of claims 1 or 3 having admixed thereto or associated in a separate container an antagonist of G-protein coupled receptors or signaling pathways.

5. The method of any one of claims 2 or 3, further comprising the administration of an antagonist of G-protein coupled receptors or signaling pathways.

6. The inhibitor of claim 4 or the method of claim 5, wherein the antagonist of G-protein coupled receptors or signaling pathways is aspirin or is an inhibitor of P2Y1, P2Y12 or PAR receptors.

7. The inhibitor of any one of claims 1, 3, 4, and 6 or the method of any one of claims 2, 3, 5 and 6, wherein the disorder related to venous or arterial thrombus formation is selected from the group consisting of myocardial infarction, stroke, ischemic stroke, pulmonary thromboembolism, peripheral artery disease (PAD), arterial thrombosis and venous thrombosis.

8. The inhibitor or the method of claim 7, wherein said stroke is ischemic stroke.

9. A method of identifying a compound suitable as a lead compound and/or as a medicament for the treatment and/or prevention of a disorder related to venous or arterial thrombus formation, comprising the steps of:
(a) determining the level of PLD1 protein or *pld1* transcript in thrombocytes;
(b) contacting said thrombocytes or thrombocytes of the same population with a test compound;
(c) determining the level of PLD1 protein or *pld1* transcript in said thrombocytes after contacting with the test compound; and
(d) comparing the level of PLD1 protein or *pld1* transcript determined in step (c) with the PLD1 protein or *pld1* transcript level determined in step (a), wherein a decrease of PLD1 protein or *pld1* transcript in step (c) as compared to step (a) indicates that the test compound is a compound suitable as a lead compound and/or as a medicament for the treatment and/or prevention of a disorder related to venous or arterial thrombus formation.

10. A method of identifying a compound suitable as a lead compound and/or as a medicament for the treatment and/or prevention of a disorder related to venous or arterial thrombus formation, comprising the steps of:
(a) determining the level of PLD1-mediated integrin αllbβ3 activation in thrombocytes upon stimulation of the thrombocytes with an activator of PLD1;
(b) contacting said thrombocytes or thrombocytes of the same population with a test compound;
(c) determining the level of PLD1-mediated integrin αIIbβ3 activation in thrombocytes upon stimulation of the thrombocytes with an activator of PLD1 after contacting with the test compound; and
(d) comparing the level of PLD1-mediated integrin αIIbβ3 activation in thrombocytes upon stimulation of the thrombocytes with an activator of PLD1 determined in step (c) with the level of PLD1-mediated integrin αIIbβ3 activation in thrombocytes upon stimulation of the thrombocytes with an activator of PLD1 determined in step (a), wherein a decrease in the level of PLD1-mediated integrin αIIbβ3 activation in thrombocytes in step (c) as compared to step (a) indicates that the test compound is a compound suitable as a lead compound and/or as a medicament for the treatment and/or prevention of a disorder related to venous or arterial thrombus formation.

11. The method of claim 10, wherein the activation of integrin αIIbβ3 in thrombocytes is determined by measuring the amount of thrombocyte aggregation, the amount of fibrinogen binding or the amount of thrombocyte adhesion to von Willebrand factor under high shear flow conditions.

12. The method of claim 10 or 11, wherein the activator of PLD1 is selected from the group consisting of GPlb-V-IX, GPVI, protease activated receptors 1 and/or 4, ADP receptors P2Y1 and P2Y12, CLEC-2 and thromboxane A2 receptor.
